# EUROPEAN PATENT SPECIFICATION

(11) **EP 2 118 967 B1**
(45) Date of publication and mention of the grant of the patent: **04.05.2016**
(21) Application number: 08730003.4
(22) Date of filing: 15.02.2008
(51) Int. Cl.: H01R 4/50

(54) **ULTRAVIOLET LAMP FOR USE IN WATER PURIFIERS**
UV-LAMPE ZUR VERWENDUNG FÜR WASSERREINIGUNGSFILTER
LAMPE A RAYONNEMENT ULTRA-VIOLET DESTINEE A DES PURIFICATEURS D'EAU

(30) Priority: 15.02.2007 US 675315; 10.10.2007 US 870256
(43) Date of publication of application: 18.11.2009
(73) Proprietor: Light Sources, Inc., Orange, CT 06477 (US)
(72) Inventor: ZAYAS, Betty, Jean, Bridgeport, CT 06610 (US)
(74) Representative: Patentanwälte Bauer Vorberg Kayser
(86) International application number: PCT/US2008/054118
(87) International publication number: WO 2008/101181

(56) References cited:
- WO-A1-2006/136026
- WO-A2-2007/098163
- US-A- 2 135 267
- US-A- 4 256 989
- US-A- 5 040 993
- US-A- 5 166 527
- US-A- 5 422 487
- US-A- 5 727 963
- US-A1- 2004 247 251
- US-A1- 2006 186 782
- US-B1- 6 634 902

## Description

### Background of the Invention:

### 1. Field of the Invention

This invention generally relates to improvements in lamps, especially ultraviolet lamps used in air and water purifiers and disinfection units.

### 2. Description of Related Art

Ultraviolet air and water or other liquid purifiers are known for disinfecting contaminated air or water or other liquid for domestic, industrial, municipal, or commercial use. Such purifiers include at least one lamp for emitting ultraviolet radiation installed into a chamber over which contaminated air or water or other liquid pass to kill microorganisms therein. In conventional manner, the lamp includes two electrodes spaced apart within an elongated arc tube containing a gas, particularly noble gas with or without additives. A pair of end caps (i.e., bases) are mounted at the ends of the tube. Each electrode contains two lead wires from the lamp seal each of which, or in some instances only one, are electrically connected to respective contact(s) or terminal pin(s). The lamp is typically inserted endwise into a sleeve installed in the water, other liquid or air purifier with or without the use of an external quartz sleeve. To simplify insertion and electrical connection, the pins are conveniently mounted on one of the end caps. When the electrodes are energized by voltage from an electrical power supply, an electrical discharge is initiated in the gas between the electrodes. This discharge results from a reaction between the electrical energy, gas and mercury to produce ultraviolet radiation to be emitted from the lamp in a manner well known in the art.
WO2007/098163 A2, US 5 422 487 A, US 2006/186782 disclose a lamb base and a corresponding socket, having two stepped portions each providing a raised face, while pin connectors for electrical connections are provided in each of the raised faces. US 6,634,902 B1 discloses a lamb base and a corresponding socket according to the preamble of the independent claims.

An example of an ultraviolet lamp of the type described above is disclosed in U.S. Patent 5,166,527 (527), all of the contents of which are incorporated herein by reference. The '527 patent discloses a lamp, especially useful as an ultraviolet lamp for use in a water purifier, comprising an elongated, hollow arc tube extending along a longitudinal axis between opposite end regions. The tube contains a gas, preferably a noble gas with or without additives. A pair of electrodes is spaced apart along the longitudinal axis. The electrodes are respectively mounted within the arc tube at the end regions thereof. A pair of end caps is respectively mounted at the end regions of the arc tube. A first electrical contact or pair of electrical contacts or teiminal pins extends in mutual parallelism along the longitudinal axis and is electrically connected to one or both of the electrode lead wires. A second electrical contact or pair of electrical contacts or teiminal pins extends in mutual parallelism along the longitudinal axis and is electrically connected to one or both of the other of the electrode lead wires. Both pairs of pins are mounted on, and extend outwardly along the longitudinal axis of, one of the end caps. A wire conductor is, or two wire conductors are, located exteriorly of the tube and electrically connected to one pin or one pair of pins at one end region of the tube, as well as to the electrode at the other end region of the tube.

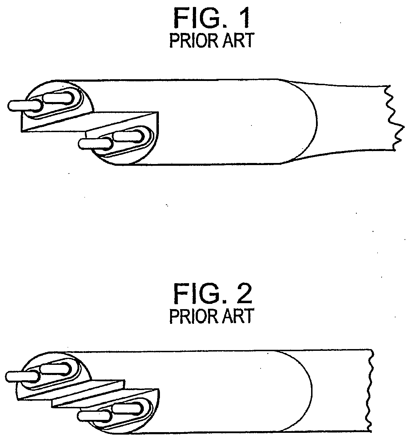

Two embodiments, of the lamp disclosed in the '527 patent are shown in prior art Figures 1 and 2. As shown in Figures 1 and 2, the pairs of pins are offset relative to each other along the longitudinal axis. This offset resists the formation of an electrical arc between the pairs of pins exteriorly of the arc tube, especially in the presence of the moisture-laden, humid environment of the water purifier.

Still referring to prior art Figures 1 and 2, the one end cap has a stepped outer end wall having a pair of wall portions lying in mutual parallelism in planes generally perpendicular to the longitudinal axis. The first pair of pins is supported on, and extends through, one of the end wall portions for a predetermined distance, and the second pair of pins is supported on, and extends through, the other of the end wall portions for the same predetermined distance. The planes of the end wall portions are spaced apart by a predetermined spacing larger than said predetermined distance in order to still further prevent electrical arcing between the pairs of pins exteriorly of the arc tube. A barrier wall internally of the one end cap physically separates the electrical connections to the two pairs of pins to resist the formation of an electrical arc between the pairs of pins interiorly of the one end cap. The barrier wall is advantageously made of the same non-conductive material as the one end cap.

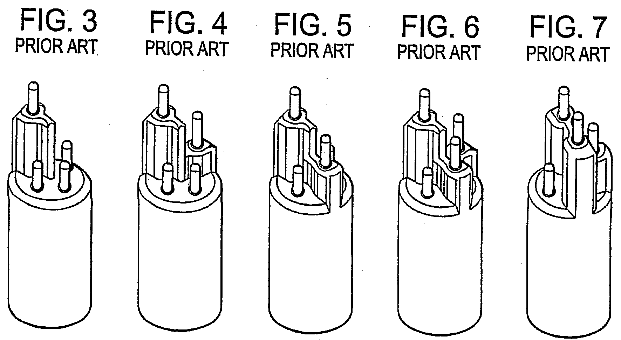

In addition to the two-tiered stepped bases of the '527 patent, other multi-tiered bases have been manufactured including those bases shown in prior art Figures 3-7.

Although well-suited for their intended purposes, there continues to be a need for improved lamp base designs, particularly ultraviolet lamps.

There are many different types of lamps that have a base or end cap that can be connected with a socket to provide electrical connections to the lamps. The end cap and socket must be constructed to permit easy replacement of the lamp while securely holding the lamp. This may be particularly important in certain applications where vibration or movement of the lamp or lamp fixture could result in unintentional separation between the lamp's end cap and the socket. Additionally, the electrical connections must remain secure.

In some applications where a multiplicity of contact pins and/or pin orientations is utilized, it is often difficult to align the contact pins to make the electrical connection necessary to operate the lamp. Often, the contact pins may become misaligned or bent due to their extension or projection from the base of the lamp, preventing their insertion into a socket. It may also be possible to insert the end cap into a socket such that the contacts are not connected with the proper terminals in the socket, resulting in improper operation of the lamp. Also, on a typical "slide into place" male/female pin connector there is no locking or twist locking and thus the pins may slide out and become disconnected easily by vibration for example.

An example of an ultraviolet lamp of the type described above is disclosed in U.S. Patent 5,166,527 ('527), which uses a stepped base with pins connector, all of the contents of which are incorporated herein by reference. The '527 patent discloses a lamp or bulb, used as an ultraviolet lamp for use in an air or water purifier, comprising an elongated, hollow arc tube extending along a longitudinal axis between opposite end regions. The tube contains a gas, preferably a noble gas with or without additives. A pair of electrodes is spaced apart along the longitudinal axis. The electrodes are respectively mounted within the arc tube at the end regions thereof. A pair of end caps is respectively mounted at the end regions of the arc tube. A first electrical contact or pair of electrical contacts or terminal pins extends in mutual parallelism along the longitudinal axis and is electrically connected to one or both of the electrode lead wires. A second electrical contact or pair of electrical contacts or terminal pins extends in mutual parallelism along the longitudinal axis and is electrically connected to one or both of the other of the electrode lead wires. Both pairs of pins are mounted on, and extend outwardly along the longitudinal axis of, one of the end caps. A wire conductor is, or two wire conductors are, located exteriorly of the tube and electrically connected to one pin or one pair of pins at one end region of the tube, as well as to the electrode at the other end region of the tube. This design is mainly designed to prevent electrical arcing and does not lock in place.

As noted in WO/2006/ 136026 to Elku et al. which is a variation of the slide-on pin connector above, a potential problem with this approach is that in many applications, the radiation lamp is immersed in a flow of water and turbulence created within that water treatment system invariably imparts a vibratory motion to the lamps. This frequently results in the lamp being vibrated or shaken loose of its electrical connection base or socket thereby causing the lamp to be rendered completely or intermittently inoperative. When such an event occurs, the water being treated may not be fully disinfected. The prior art has attempted to address this problem by using a relatively complicated mechanical connection (e.g., a so-called "push-and-twist" connection) to secure the lamp to the connection base. See, for example, United States patent 5,422,487 to Sauska et al. and United States patent 6,884,103 to Kovacs. The potential problem with these approaches is the complexity of the mechanical connection between the lamp and the base unit requiring the use of springs, specialized connection lugs and the like. Further, a connection system which is predicated on a dual motion system such that pushing and twisting if used incorrectly for example may give rise to higher incidents of lamp breakage, electrical shock, and other damage to the lamp by field personal. Therefore, eliminating a forceful "push" necessary to deflect a heavy locking spring in a "push and twist" lock would be beneficial because the typically glass lamp would be subject to reduced force and stress.

Also, it is important that lamps of proper wattage be used for safety, heat, and fire concerns. Thus, a unique keying system that only allows lamps of proper wattage to be inserted into the base will also help safety.

Accordingly, there remains the need in the art for a safety lamp device, particularly a radiation lamp, which will provide a reliable, locking, and secure from movement, electric connection, yet be relatively inexpensive, uncomplicated, durable, rugged, and simple to implement with smooth operation and with reduced force and stress on the lamp for safety purposes. Also, a lamp that reduces the chance of electrical shock is needed for safety purposes.

Thus, there continues to be a need for improved lamp base designs, particularly ultraviolet lamps.

### Summary of the Invention:

In accordance with at least an embodiment of the present invention, a lamp base includes a cylindrical body having an end surface, a first step portion and a second step portion, a first upper pin connector provided an the first step portion, a second upper pin connector provided an the second step portion, and a first and second lower pin connector provided an the end surface.

The above-described end cap configuration for the lamp may be mated to a complimentary shaped receptacle known as a socket. The socket may be wired to a power source.

The designs will further allow for matching of the lamp and connector to a specific power supply to reduce the chance of connecting an ultraviolet lamp into an improperly matched power supply.

The lamp of this invention is preferably an ultraviolet lamp and finds particular application for use in a water, other liquid, or air purifier.

The features of the lamp base, the socket and the assembly in accordance with the invention are defined in claims 1, 7, and 13 respectively.

Preferred embodiments are defined in the claims 2-6, 8-12 and 14-16.

### Brief Description of the Drawings:

Embodiments will now be described, by way of example only, with reference to the accompanying drawings which are meant to be exemplary, not limiting, and wherein like elements are numbered alike in several Figures, in which:
FIGURES 1-7 are respective views of end caps used in ultraviolet lamps in accordance with the prior art;
FIGURE 8 is a cross-sectional view along the longitudinal axis of a water purifier employing the ultraviolet lamp;
FIGURE 9 is a cross-sectional elevation view along the line 9-9 of FIGURE 8;
FIGURE 10 is a perspective elevation view of a first embodiment of an end cap for a lamp;
FIGURE 10A is a cross-sectional elevation view along the line 10A-10A of FIGURE 10;
FIGURES 11-14 are perspective elevation views of second, third, fourth and fifth embodiments of end caps;
FIGURES 15-19 are perspective elevation views of sockets for the end caps of FIGURES 10-14, respectively;
FIGURES 20-24 are perspective elevation views of additional embodiments of end caps having female recesses; and
FIGURES 25-29 are perspective elevation views of sockets for the end caps of FIGURES 20-24, respectively.
FIGURES 30-31 are perspective views of an embodiment of a socket useable with the lamp base shown in Figs. 38-45.
FIGURES 32-35 are side views of the embodiment shown in Figs. 30-31.
FIGURE 36 is a top plan view of the embodiment shown in Figs. 30-35
FIGURE 37 is a bottom plan view of the embodiment shown in Fig. 30-35.
FIGURES 38-39 are perspective views of a lamp base useable with the socket of shown in Figures 30-37.
FIGURES 40-43 are side views of the base shown in Figs. 38-39.
FIGURE 44 is a top plan view of the base shown in Figs. 38-43
FIGURE 45 is a bottom plan view of the base shown in Figs. 38-44.

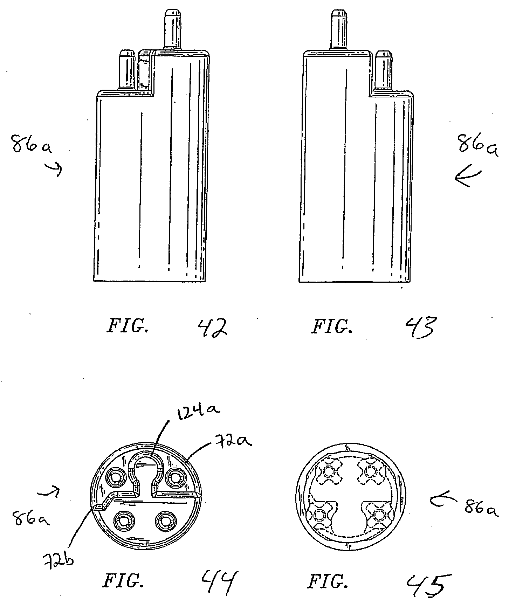

Figures 1-45 do not form part of the invention as claimed.

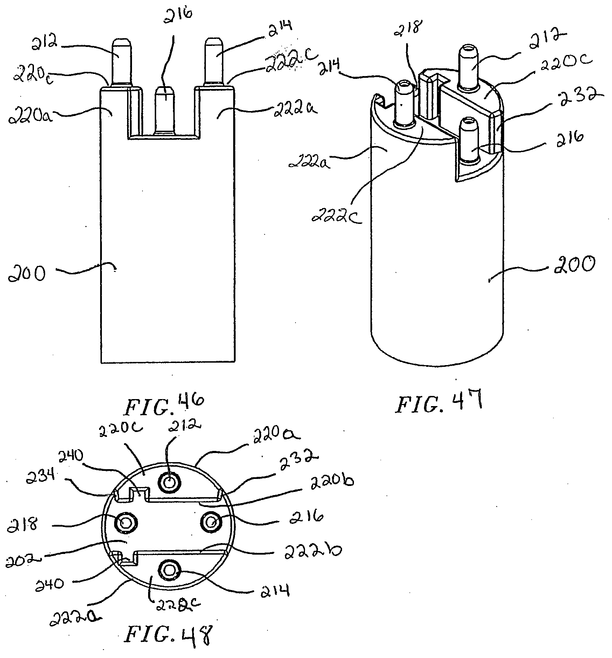

FIGURES 46-48 are various views of a lamp base according to at least an embodiment of the present invention.

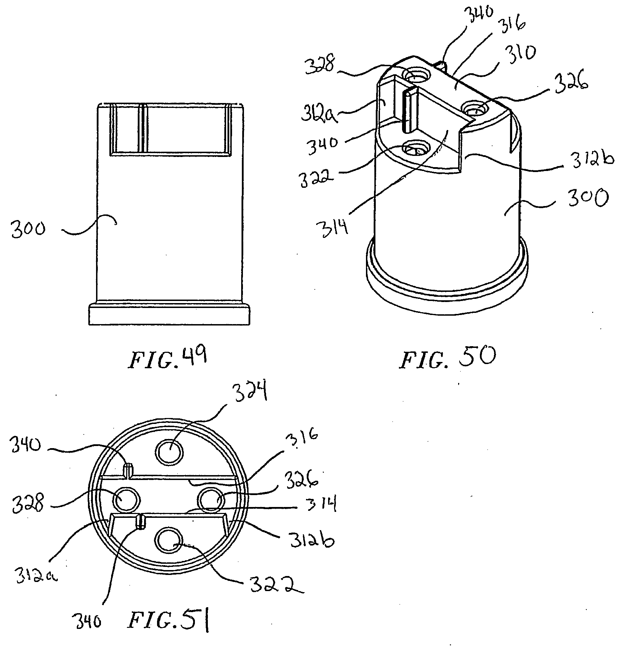

FIGURES 49-51 are various views of a socket according to at least an embodiment of the present invention.

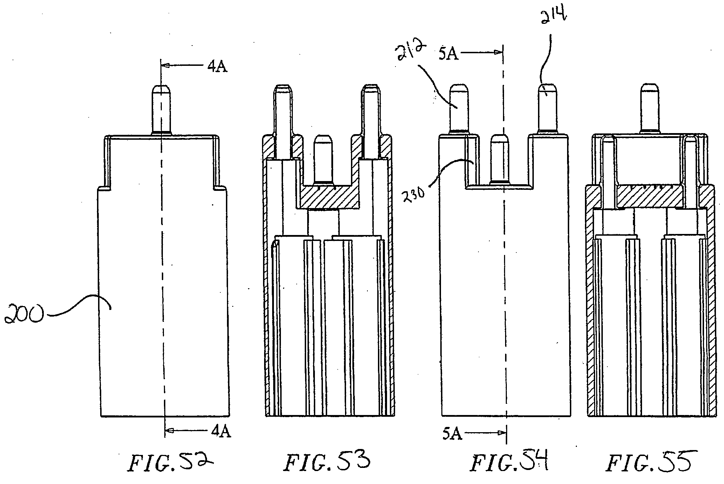

FIGURES 52-55 are various views of a lamp base according to at least an embodiment of the present invention.

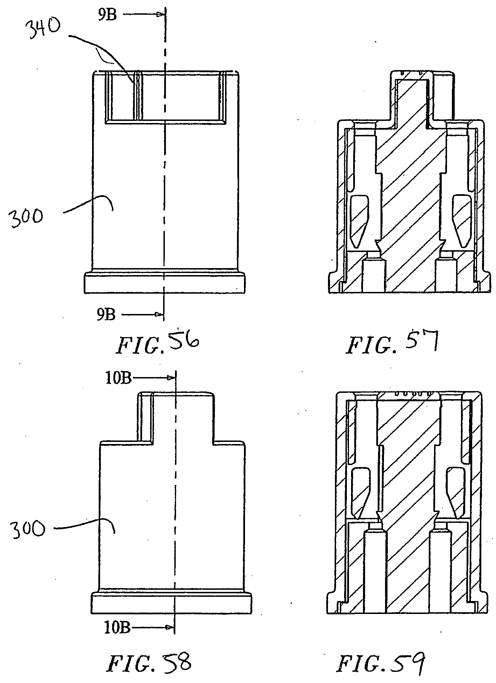

FIGURES 56-59 are various views of a socket according to at least an embodiment of the present invention.

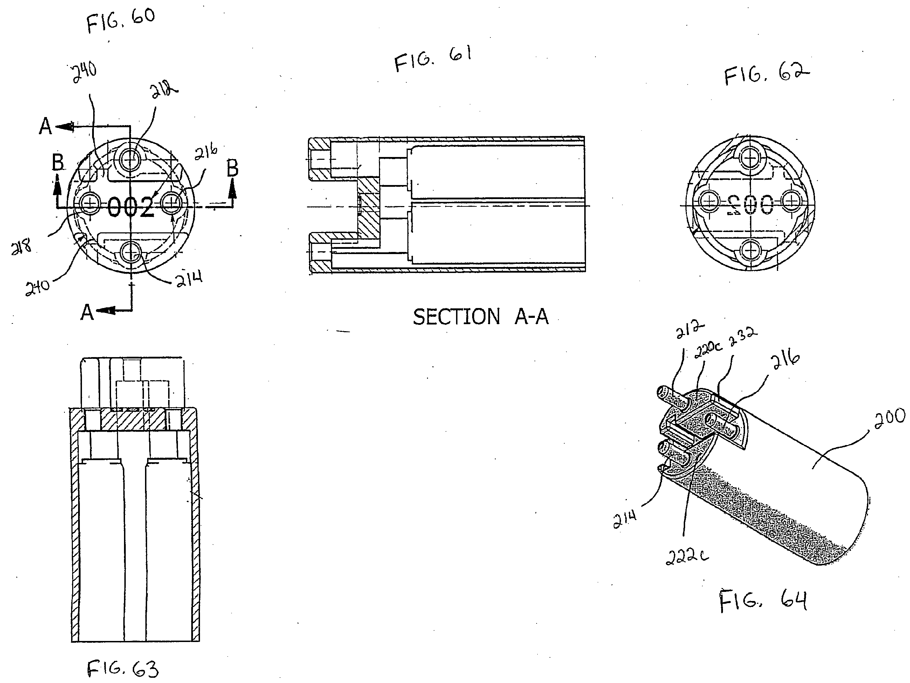

FIGURES 60-64 are various views of a lamp base according to at least an embodiment of the present invention.

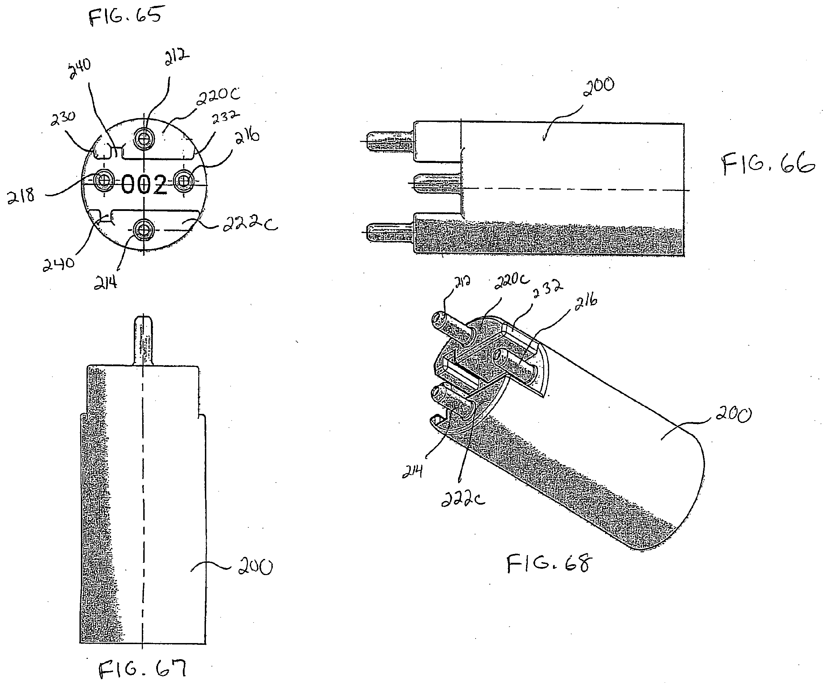

FIGURES 65-68 are various views of a lamp base according to at least an embodiment of the present invention.

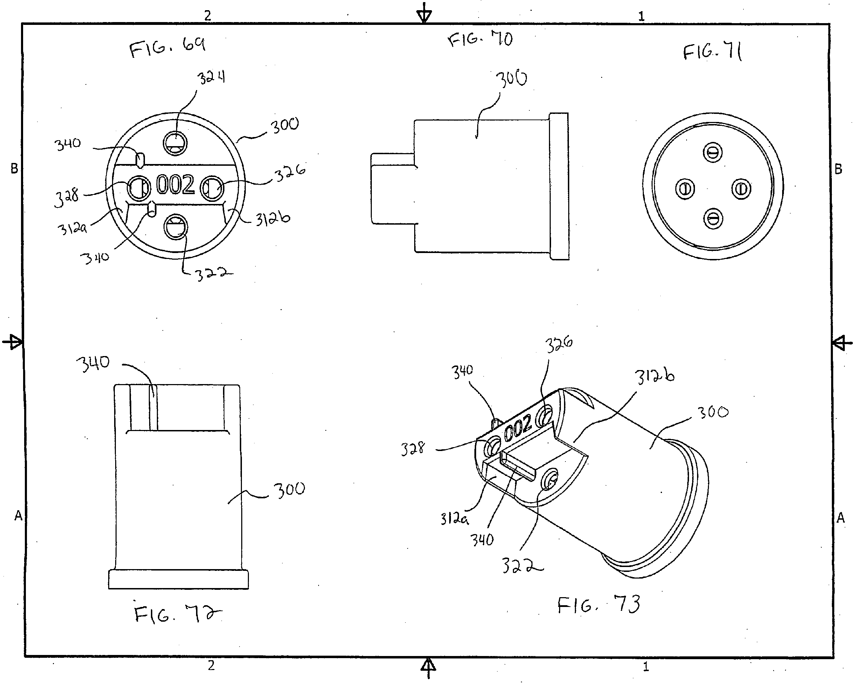

FIGURES 69-73 are various views of a socket according to at least an embodiment of the present invention.

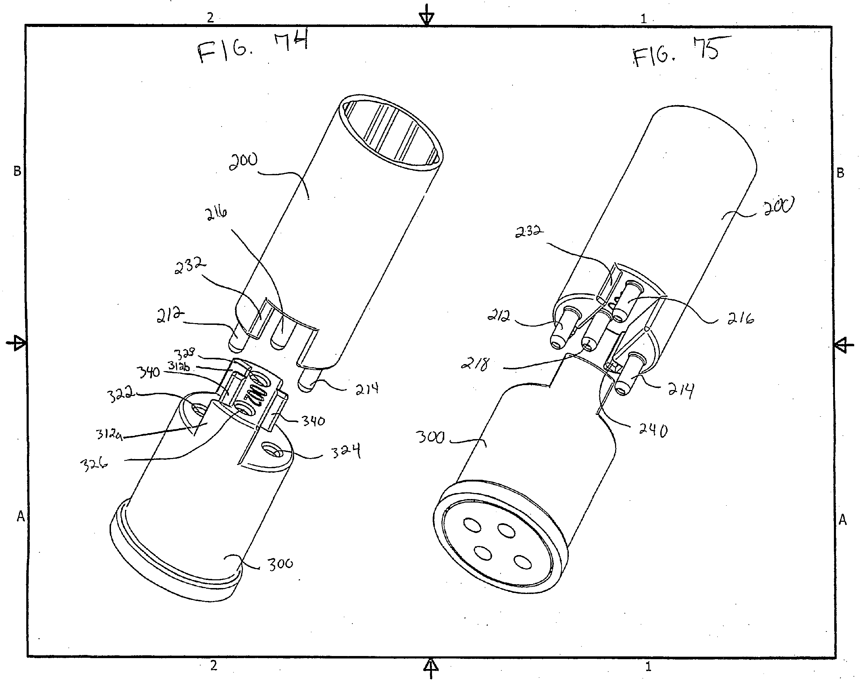

FIGURES 74-75 are perspective views of a lamp base and socket assembly according to at least an embodiment of the present invention.

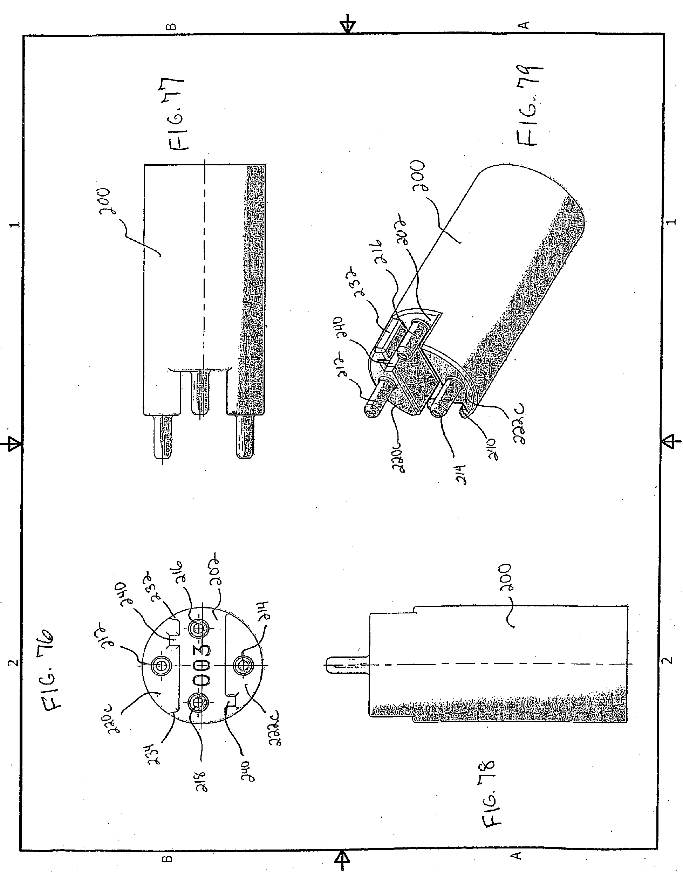

FIGURES 76-79 are various views of a lamp base according to at least an embodiment of the present invention.

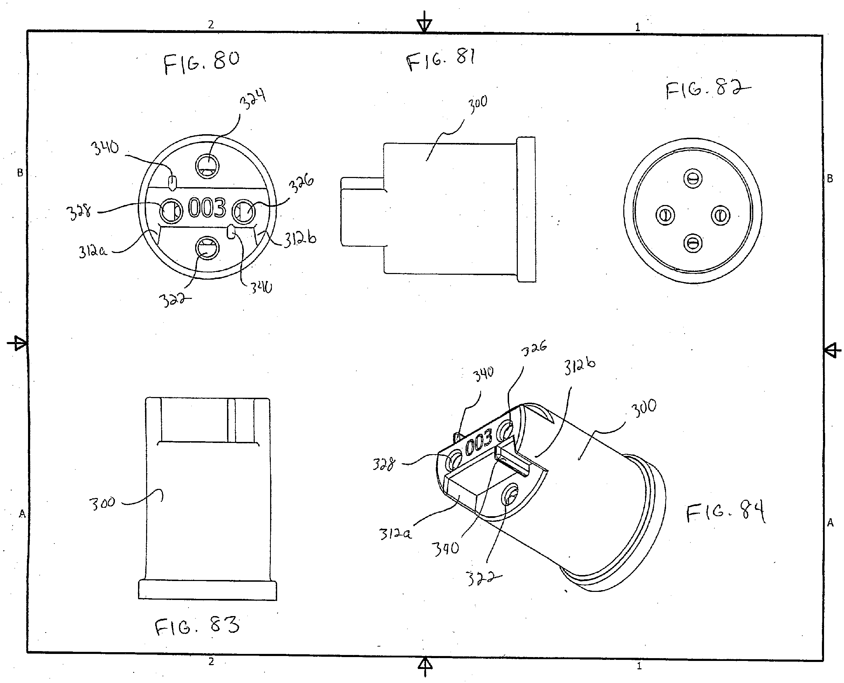

FIGURES 80-84 are various views of a socket according to at least an embodiment of the present invention.

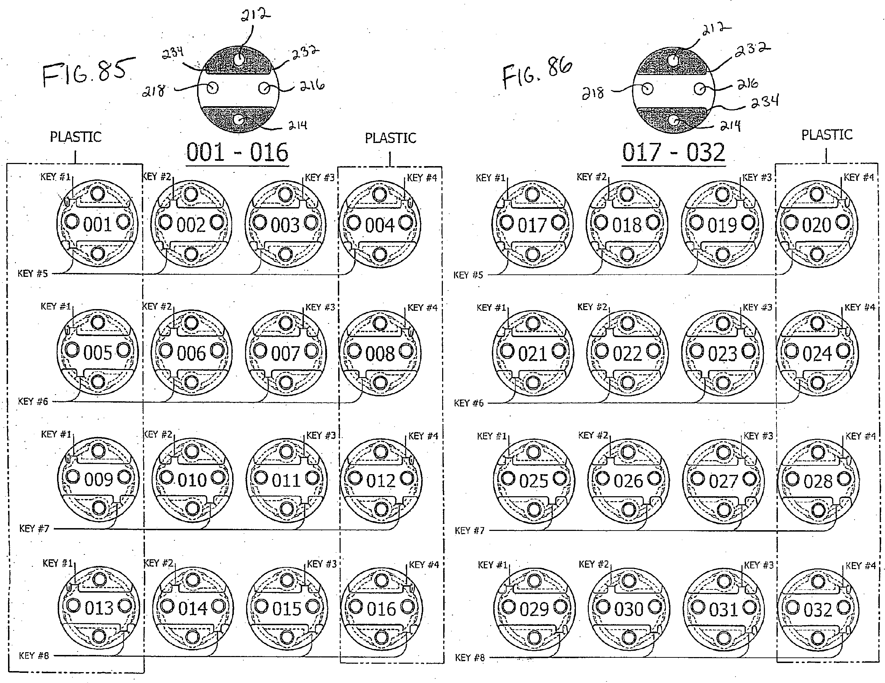

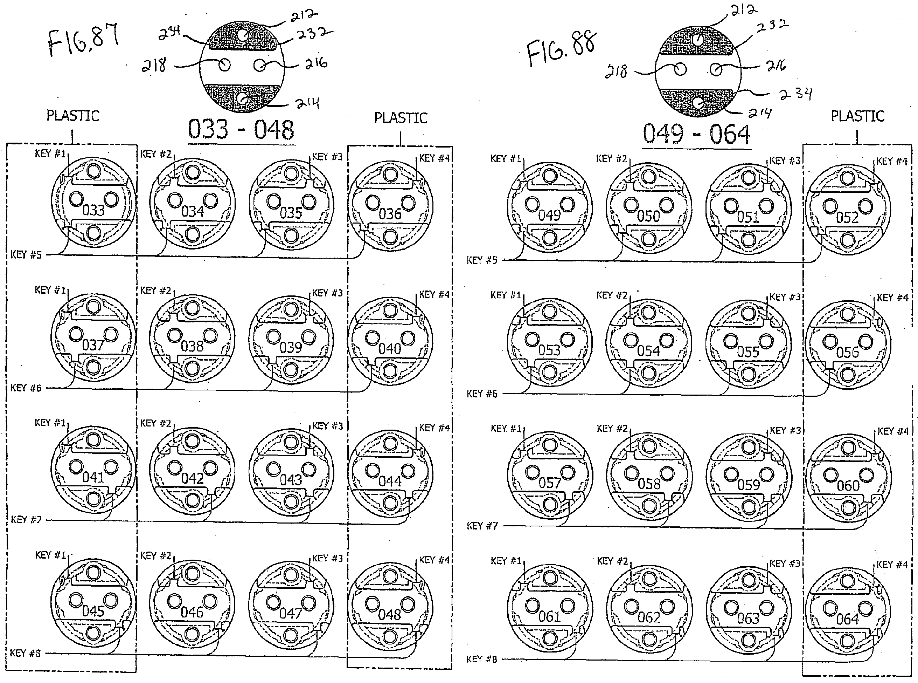

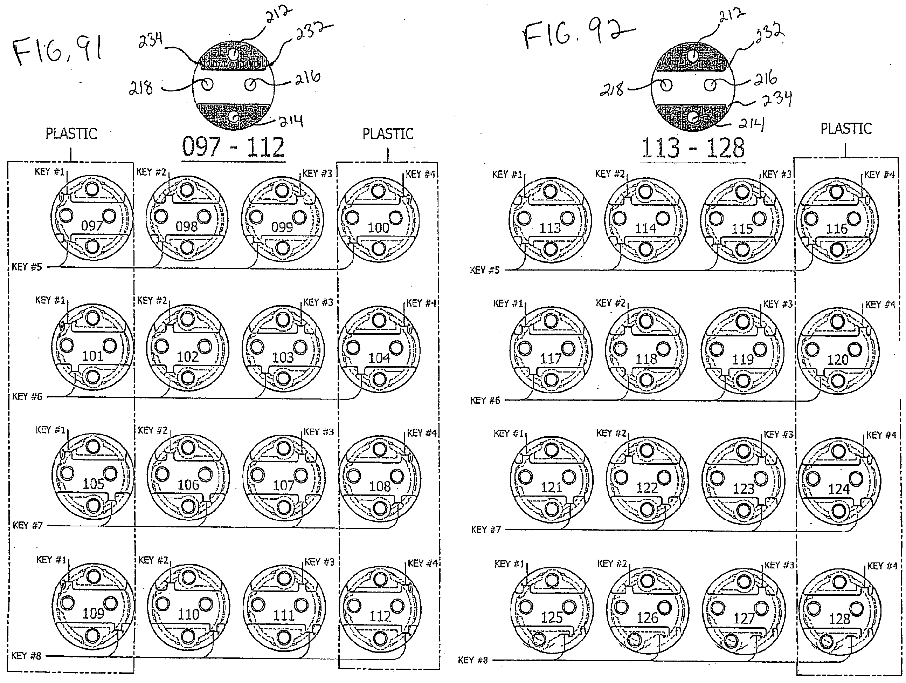

FIGURES 85-92 are plan views show various possible configurations of pin connectors, truncated parts, and keys according to at least some embodiments of the present invention.

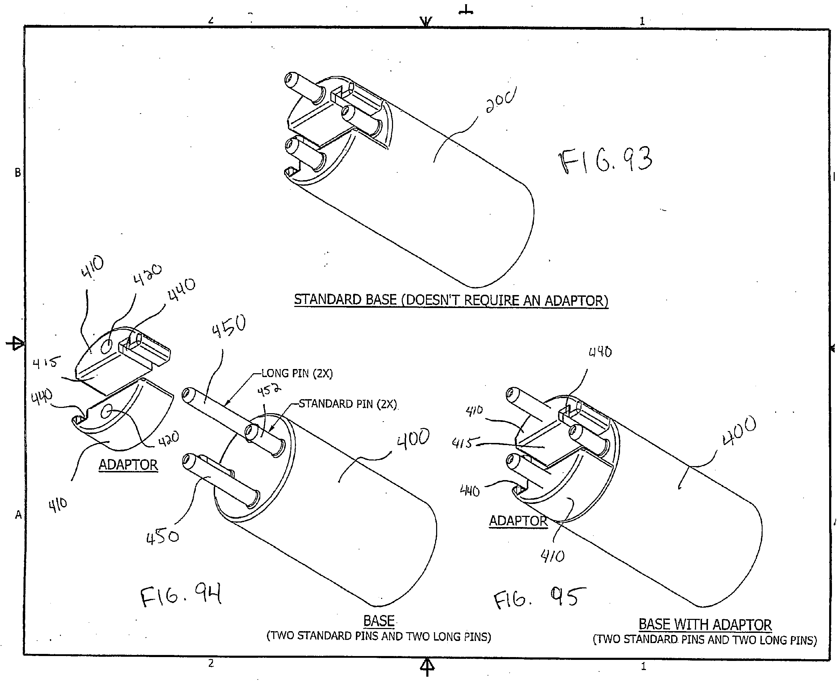

FIGURES 93 shows a lamp base according to at least an embodiment of the present invention.
FIGURE 94 is an exploded perspective view of a lamp base and an adapter according to at least an embodiment of the present invention.
FIGURE 95 is an assembled perspective view of a lamp base and an adapter according to at least an embodiment of the present invention.

### Detailed Description of the Preferred Embodiments:

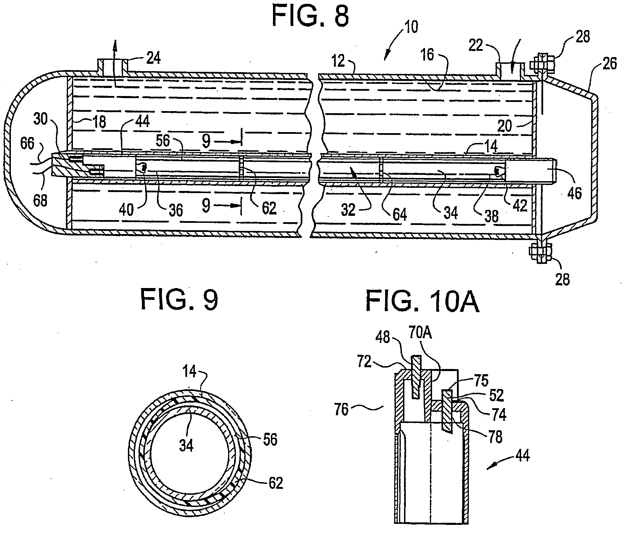

Referring now to FIGURE 8 of the drawings, reference numeral 10 generally identifies a water purifier having a cylindrical housing 12 extending along a longitudinal axis. A hollow, axially-extending sleeve 14 is centrally mounted within the housing 12 between apertured support walls 18, 20 spaced apart along the longitudinal axis. The support walls 18, 20, together with the interior wall of the housing 12 and the exterior wall of the sleeve 14, bound an interior space 16. A water inlet 22 admits pressurized water to be purified into the space 16. A water outlet 24 discharges the purified water from the space 16. The space 16 serves as a flow-through passage for the water contained therein which, during the course of its flow, is exposed to ultraviolet radiation as described below.

A removable cover 26 overlies the support wall 18 at one end of the housing 12. The cover 26 is detachably coupled to the housing 12 by a set of threaded fasteners 28 to permit access to the interior of the sleeve 14 through the aperture of the support wall 18. An electrical socket 30 is removably mounted within the aperture of the support wall 18. An ultraviolet lamp 32 is slidably inserted into the interior of the sleeve 14 through the aperture of the support wall 18. An electrical connection, as described below, is made with the socket 30. In operation, the lamp 32 emits ultraviolet radiation of sufficient intensity to kill microorganisms in the water, other liquid or air contained in the space 16 to purify the same for domestic and commercial applications.

The lamp 32 includes an elongated, hollow, sealed, arc tube 34 constituted of a light-transmissive material, e.g. silica quartz or other ultraviolet transmitting glass tube. The tube 34 has opposite end regions 36,38 spaced apart along the longitudinal axis. A gas, preferably mercury vapor with or without additives, is sealingly contained within the tube.

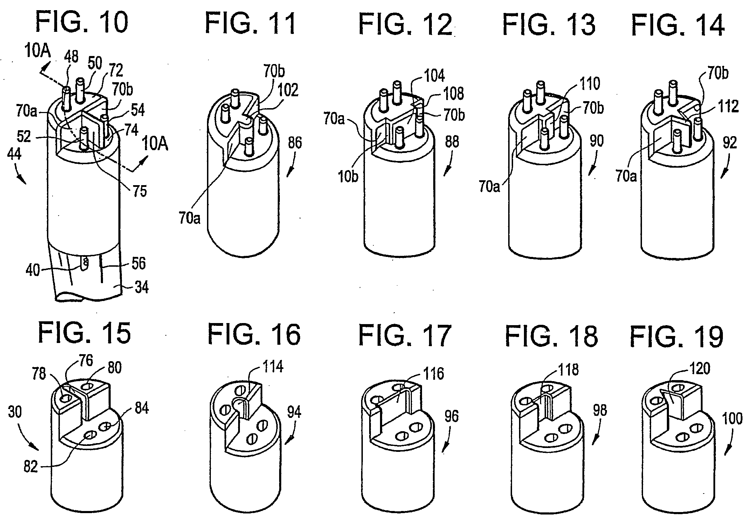

A pair of electrodes 40, 42 is respectively mounted within the tube at the end regions 36, 38. A pair of end caps 44, 46 constituted of a non-conducting material, e.g. ceramic, is respectively mounted at the end regions 36, 38 over the sealed end regions of the tube. Each end cap has a bore having a closed base against which a sealed end region of the tube abuts when the sealed end region is inserted fully into a respective end cap. End cap 44 (best show in FIGURES 10 and 10A) also has a barrier wall 70a, 70b, extending between semi-circular bases 72, 74 such that bases 72, 74 are stepped with base 72 being the upper or distal base and base 74 being the lower or proximal base. A male member 75 extends laterally from barrier wall 70a, 70b, along lower base 74. Member 75 is centrally positioned along wall 70a, 70b thus splitting the wall into the two sections 70a and 70b. Member 75 has a height which is equal to the distance between respective bases 72 and 74. Member 75 also acts to divide lower base 74 into two symmetrical sections (each comprising an approximately 90 degree quadrant).

Still referring to FIGURE 10, a first pair of electrical contacts or terminal pins 48, 50 extends in mutual parallelism along the longitudinal axis, and is mounted on, and extends outwardly of, the end cap 44 (through base 72). A second pair of electrical contacts or terminal pins 52, 54 also extends in mutual parallelism along the longitudinal axis, and is also mounted on, and extends outwardly of, the same end cap 44 (through base 74). Pins 52 and 54 are separated from one another by member 75. The pins 48, 50 extend into interior compartment 76, and the pins 52, 54 extend into compartment 78 (see FIGURE 10A).

The pins 48, 50 are electrically connected within end cap 44 to one end of a wire conductor 56. The conductor 56 is located exteriorly of the tube 34, and extends along the longitudinal axis to the opposite end cap 46 wherein the other end of the conductor 56 is connected to the electrode 42. The pins 52, 54 are electrically connected within the end cap 44 to the electrode 40. The barrier wall 70a, 70b physically separates the pairs of pins.

Thus, as shown in FIGURE 10, the end cap 44 has a stepped, outer end wall having a pair of semi-circular end wall portions 72, 74 lying in mutual parallelism in planes generally perpendicular to the longitudinal axis. As best shown in FIGURE 15, the socket 30 has a complementary stepped, outer end wall together with a centrally located recess 76. During mating, the pins 48, 50, 52, 54 will be received into respective sockets 78, 80, 82, 84 while member 75 is received into mating recess 76.

A set of centering rings 62, 64, preferably constituted of a synthetic plastic material, is located on tube 34, being placed thereon before the end caps are attached to the end regions of the tube 34. The rings 62, 64 coaxially surround the tube 34 and frictionally engage and support the tube, and assist in centering the tube within sleeve 14.

Referring now to FIGURES 11-14, additional embodiments of the end cap depicted in FIGURE 10 are shown. All of these end caps have a similar configuration to that shown in FIGURE 10 including a pair of parallel but longitudinally displaced semi-circular bases 72, 74 having a barrier wall 70a, 70b, extending therebetween so as to form a stepped base. In addition, a male member or extension extends centrally outwardly from barrier wall 70a, 70b. While this male extension was in the form of a relatively thin wall 75 in the end cap 44 of FIGURE 10, the male extension takes on a differing geometric shape in the various embodiments shown in FIGURES 11-14. More specifically, the end cap 86 in FIGURE 11 has a rounded arcuate shape (e.g., V-shaped, semi-circular shaped) extension 102 extending between barrier wall sections 70a and 70b. The end cap 88 in FIGURE 12 has an extension 104 in the shape of a rectangular box which includes a pair of opposed sidewalls 106, 108 which extend outwardly from and perpendicularly to respective barrier wall sections 70a and 70b. The end cap 90 of FIGURE 13 also has a box like extension 110 which is similar to extension 104 (in FIGURE 12); however, extension 112 has a more square cross-section relative to the rectangular cross-section of extension 104. Thus, the barrier wall sections 70a and 70b in FIGURE 13 are relatively larger than the respective barrier wall sections 70a and 70b in FIGURE 12. End cap 92 in FIGURE 14 is a V-shaped triangular shape extension member 112 which forms the divider between barrier wall sections 70a and 70b.

Significantly, all of the additional embodiments of FIGURES 11-14 have in common the feature of FIGURE 10 wherein a male member extends outwardly from barrier wall 70a, 70b, and is coextensive with stepped (that is longitudinally displaced) base sections 72, 74. It will be appreciated that in accordance with this invention, the male member may have any suitable configuration besides those shown in FIGURES 10-14 and that such configuration may be symmetrical, asymmetrical, spherical, conical, aspheric or any other desired shapes.

As in the receptacle 30 shown in FIGURE 15 which receives the end cap 44 in FIGURE 10, the end caps of the embodiments of FIGURES 11-14 have similarly complimentary shaped respective receptacles 94, 96, 98 and 100 as shown in FIGURES 16-19, respectively. Thus, receptacle 94 of FIGURE 16 has a rounded arcuate shaped recess 114 for receiving and mating with rounded or U-shaped extension 102 in FIGURE 11. Similarly, receptacle 96 has a rectangular shaped recess 116 which is sized and configured to mate with and be received by rectangular extension 104 in FIGURE 12. Receptacle 98 in FIGURE 18 has a square shaped recess 118 for mating with and being received by square shaped extension 110 in FIGURE 13 while receptacle 100 of FIGURE 19 has a V-shaped recess 120 for mating with and being received by V-shaped extension 112 in FIGURE 14. Again, it will be appreciated that the receptacles of FIGURES 15-19 may include a recess having any desired shape, so long as the shape is complimentary to, and can be received by, the shape of the extensions in the mating end cap.

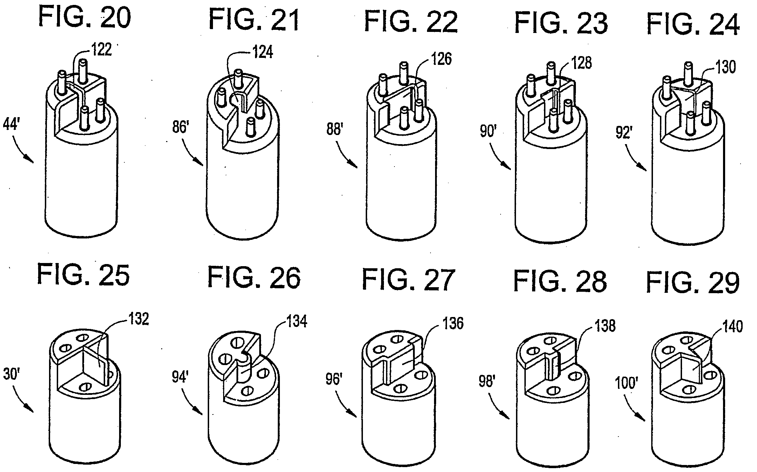

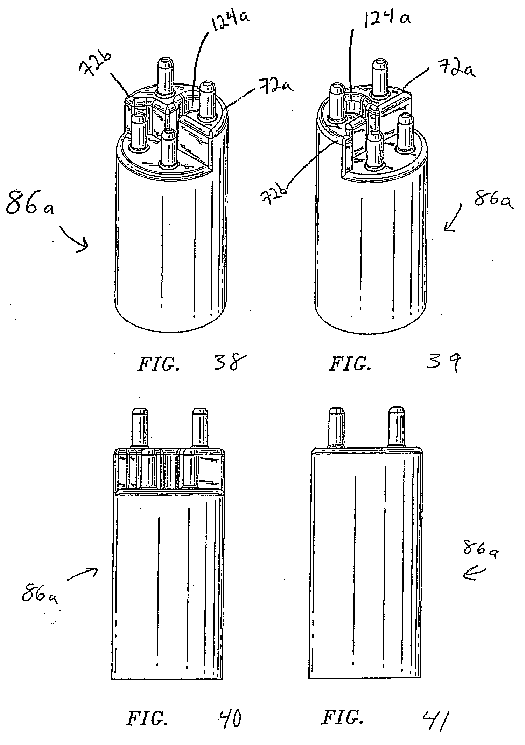

In still another alternative embodiment, it will be appreciated that the male extension in the plug ended lamp and the female recess in the complimentary receptacle may be reversed such that the lamp cap will exhibit the recess while the receptacle will exhibit the complimentary male extension for mating with the recess in the cap. Such alternative configurations are shown in the end caps of FIGURES 20-24 and also in FIGURES. 38-45. The end caps of FIGURES 20-24 and 38-45 are similar to the respective end caps shown in FIGURES 10-14 with the only difference being that the male extension has been substituted with the female recess in FIGURES 20-24. Thus, end cap 44' includes a narrow recess 122 (similar to the recess 76 shown in FIGURE 15), end cap 86' and 86a include an arcuate shaped recess 124, 124a (similar to the recess 114 in FIGURE 16), end cap 88' includes a rectangularly shaped recess 126 (similar to the recess 116 in FIGURE 17), end cap 90' of FIGURE 23 includes a square shaped recess 128 (similar to the recess 118 in FIGURE 18) and end cap 92' of FIGURE 24 includes a V-shaped recess 130 (similar to the V-shaped recess 120 of FIGURE 19).

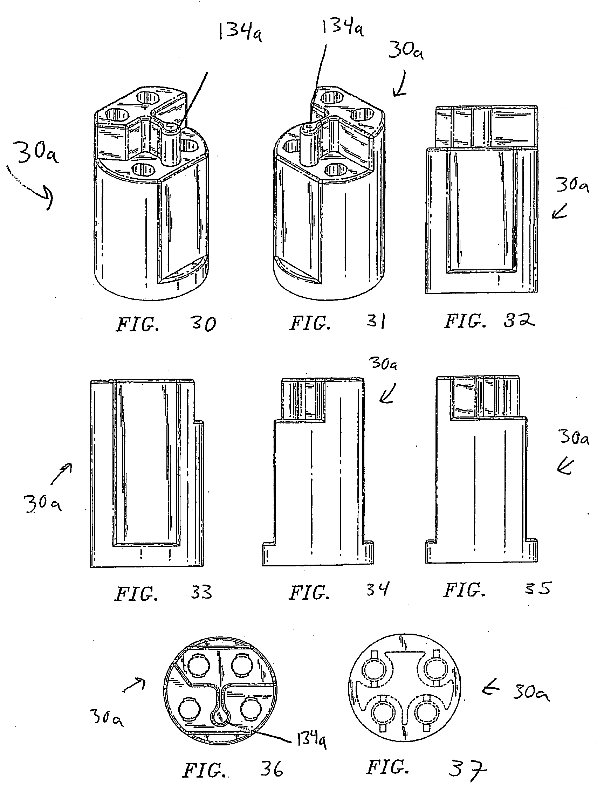

Similarly, with reference to FIGURES 25-29 and 30-37, receptacles or bases are shown which are configured to mate with the end caps of FIGURES 20-24 and 38-45, respectively. Thus, receptacle 30' of FIGURE 25 includes male extension 132 which is sized and configured to be received by recess 122 in FIGURE 20. Receptacle 30a of FIGURES 30-37 includes male extension 134a which is sized and configured to be received by recess 124a in FIGURE 38. Fig. 38 also shows how semi-circular bases 72a may contain an asymmetrical section 72b which may be added to help ensure that unauthorized parts are unable to be used. This is a safety feature because improper wattage lamps or improper lamp designs may commonly be attempted to be substituted so the asymmetrical section 72b helps to prevent this unsafe occurrence. The sockets shown on FIGURES 30-37 may be used with the corresponding end caps or bases shown in FIGURES 38-45 or the designs may be exchanged with each other, i.e., the end cap may look like the receptacle or base or vice versa. Receptacle 94' of FIGURE 26 includes arcuate shaped male extension 134 which is sized and configured to be received by arcuate recess 124 in FIGURE 21. Similarly, receptacle 96' of FIGURE 27 includes a rectangular extension 136 which is sized and configured to be received by rectangular recess 126 of FIGURE 22, receptacle 98' of FIGURE 28 includes a square shaped extension 138 which is sized and configured to be received by squared shaped recess 128 in FIGURE 23 and receptacle 100' of FIGURE 29 includes a triangular shaped extension 140 which is sized and configured to be received by rectangular shaped recess 130 in FIGURE 24.

It will be appreciated that all of the embodiments of the present invention represent an advance over the stepped cap configuration of the aforementioned '527 patent in that the use of the extension (or alternatively the recess) positioned centrally along the barrier wall will provide improved interlocking between the lamp and the receptacle and will also ensure proper orientation of the respective pins within the recesses of the receptacle.

In operation, the cover 26 is removed, and a lamp 32 is inserted into the interior of the sleeve 14 via the aperture in the support wall 18. After insertion, the socket 30 is connected to the pins 48, 50, 52, 54. Then the cover 26 is installed on the housing 12. Electrical wires 66,68 extend exteriorly of the socket to a non-illustrated electrical power supply. A voltage difference across the wires 66,68 is applied to both electrodes 40, 42 causing an electrical discharge within the tube. This discharge causes ultraviolet radiation to be emitted. This radiation passes through the light-transmissive wall of the sleeve 14 to irradiate the water contained in the space 16.

The aforementioned barrier wall 70a, 70b prevents arcing interiorly of the end cap 44 by physically separating the electrical connections between the pins 48, 50 and the wire conductor, on the one hand, and the electrical connections between the pins 52, 54 and the electrode 40, on the other hand. The pairs of pins are thus effectively isolated.

It will be understood that each of the elements described above, or two or more together, also may find a useful application in other types of constructions differing from the types described above.

In accordance with the invention, a lamp base 200-includes at least a first step portion and a second step portion, as seen in Figures 46-48.

For example, Figures 46-48 illustrate various views of a lamp base 200 according to at least an embodiment of the present invention. The lamp base 200 is generally cylindrical in shape and has an end surface 202. Additionally, a first step portion and a second step portion may extend outward from end surface 202 of lamp base 200.

As further seen in Figures 46-48, the first step portion includes a curved face 220a, a flat face 220b, and a raised face 220c. Similarly, the second step portion includes a curved face 222a, a flat face 222b and a raised face 222c. Curved faces 220a, 222a and flat faces 220b, 222b extend perpendicular to the end surface. Raised faces 220c, 222c are perpendicular to curved faces 220a, 222a and flat faces 220b, 222b, respectively.

Lamp base 200 also includes a number of pin connectors. For example, as seen in Figures 47-48 (see also Figure 75), a first upper pin connector 212 extends from the raised face 220c of the first step portion, a second upper pin connector 214 extends from the raised face 222c of the second step portion, and a first lower pin connector 216 and a second lower pin connector 218 extend from the end surface.

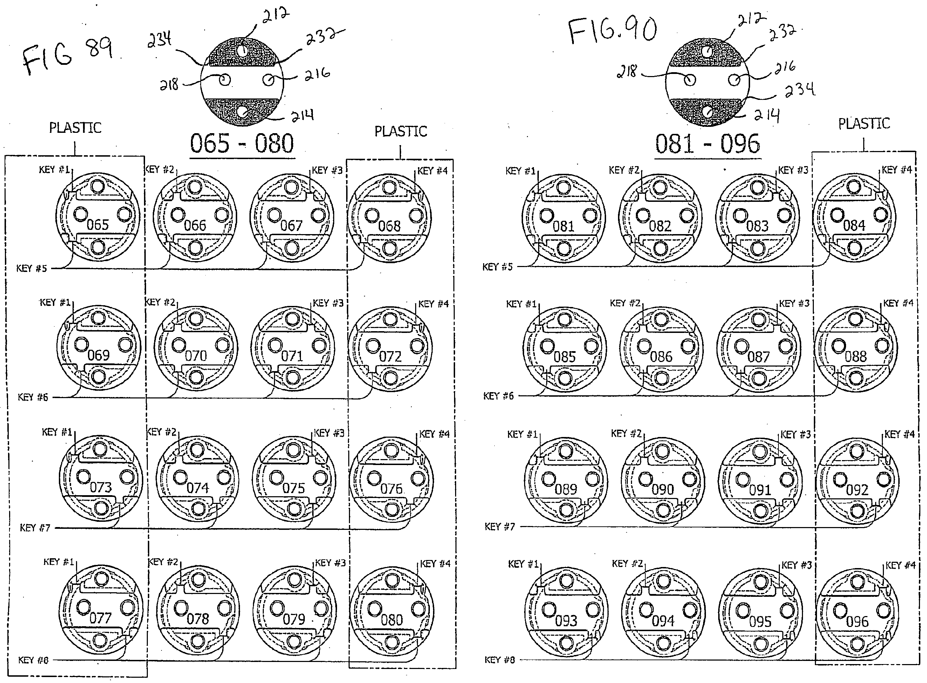

The first and second lower pin connectors 216, 218 are arranged in a variety of configurations. For example, the first and second lower pin connectors 216, 218 are arranged symmetrically with respect to a center of the end surface, as seen in Figures 85-88. Additionally, the first and second lower pin connectors 216, 218 are arranged asymmetrically with respect to a center of the end surface, as seen in Figures 89-92. These arrangements of the lower pin connectors 216, 218 are helpful in ensuring proper alignment when a lamp base is coupled with a socket, as explained in detail below.

Additionally, key recesses 240 are provided an the lamp base 200 to help in alignment when coupling with a socket, as explained in more detail below. As seen in Figure 48, for example, key recesses 240 are provided in the flat faces 220b, 222b of the first and second step portions.

There are many different possible configurations of key recesses, as seen in Figure 85, for example. Figure 85 shows how a key recess can be found at one of at least four positions (key #1, key #2, key #3, key #4) in the flat face of the step portion.
Additionally, Figure 85 shows how a key recess can be found at one of at least four positions (key #5, key #6, key #7, key #8). It is important to note that the keys can be placed independent from each other, and thus many possible combinations of key positions can be achieved. It will also be appreciated that the key positions are not limited to only the positions shown in Figure 85, and that other suitable positions are also possible.

As seen in Figure 48, the first step portion also includes a first truncated portion 232 and a second truncated portion 234. When compared with the second step portion, truncated portions 232, 234 are flat surfaces where it appears that the step portion has been truncated, or "cut off." In at least the embodiment shown in Figure 48, the truncated portions 232, 234 are provided at a first and second end of the first step portion. However, other configurations are possible. For example, as seen in Figure 86, the first truncated portion 232 may be provided at a first end of a first step portion, and a second truncated portion 234 may be provided at a first end of a second truncated portion. The truncated portions help to ensure proper alignment when a lamp base is coupled with a socket, as explained in more detail below.

Figures 49-51 illustrate at least one possible embodiment of a socket 300. The socket 300 is generally cylindrical in shape and has an end surface. Additionally, a central step portion 310 extends perpendicular to the end surface of socket 300. Central step portion 310 may include a first flat face 314 extending perpendicular to the end surface and a second flat face 316 extending perpendicular to the end surface.

Additionally, as seen in Figures 50 and 51, socket 300 includes a first upper connector 326 provided within a recess formed in the central step portion 310 and a second upper connector 328 provided within a recess formed in the central step portion 310. Socket 300 also includes a first lower connector 322 provided within a recess formed in the end surface, and a second lower connector 324 provided within a recess formed in the end surface. The first and second upper connectors 326, 328 may be positioned either symmetrically or asymmetrically with respect to a center of the end surface of the socket 300.

Figures 50-51 also illustrate that the socket 300 includes key protrusions 340 protruding out from the first flat face 314 and the second flat face 316. These key protrusions 340 can be positioned in a wide variety of configurations, complementing the wide variety of configurations possible for key recesses 240.

Additionally, Figures 50-51 show that socket 300 also includes a first tapered portion 312a and a second tapered portion 312b. The tapered portions 312a, 312b extend out from the first and second ends of first flat face 314, as seen in Figure 51, for example, or a first tapered portion 312a extends out from a first end of first flat face 314 while the second tapered portion 312b extends out from a first end of second flat face 316.

Figures 52-73 and 76-93 show various additional views of at least some possible embodiments and configurations of lamp bases 200 and sockets 300.

Figures 74 and 75 show how a lamp base 200 and a socket 300 according to at least an embodiment of the present invention can couple with each other. Lamp base 200 and socket 300 are structured such that first upper pin connector 212 couples with first lower connector 322, second upper pin connector 214 couples with second lower pin connector 324, first lower pin connector 216 couples with first upper connector 326, and second lower pin connector 218 couples with second upper connector 328.

Additionally, it is seen in Figures 74 and 75 that the key recesses 240 of lamp base 200 are structured to align with the key protrusions 340 of the socket 300 when the lamp base and the socket are coupled. In other words, when lamp base 200 is coupled with socket 300 in the proper alignment, key protrusions 340 will slide into key recesses 240. If lamp base 200 is misaligned with socket 300, then the key protrusions 340 will not align with the key recess 240.

Additionally, as another method to ensure proper alignment, it is seen from Figures 74 and 75 that first tapered portion 312a will align with first truncated portion 232, and second tapered portion 312b will align with the second truncated portion 234.

As noted above, there are many alignment features designed to ensure that the lamp base 200 is properly aligned with socket 300 when they are coupled, for example, the key protrusions 340 and key recesses 240 and the truncated portions 232, 234 and tapered portions 312a, 312b. These alignment features result in significant and non-trivial benefits over conventional devices.

For example, it will be appreciated that the alignment features described above can ensure that there is only one possible way for a lamp base to fit into the socket. In other words, the alignment features ensure that the first upper pin connector 212 will always couple with the first lower connector 322, the second upper pin connector 214 will always couple with the second lower connector 324, etc. This is an important safety feature because it prevents the pin connectors from mistakenly being connected to the wrong polarity of a power source, for example, which could damage the lamp. Thus, the alignment features described above can help to prevent damage to lamps by ensuring proper coupling.

Additionally, the alignment features described above can help to ensure that a lamp is only coupled with an appropriate socket. For example, an appropriate socket may have a given configuration of key protrusions 340 and/or tapered portions 312a, 312b, and unless the lamp base is a properly corresponding lamp base that has complementary key recesses 240 and/or truncated portions 232, 234, the lamp base cannot be coupled to the socket.

Additionally, these features provide important safety benefits as well. For example, if connector pins are improperly connected to the wrong polarities, sparks can be generated that pose a fire risk, or the user may be exposed to electric shock. Each of the alignment features above helps to ensure proper coupling of lamp bases and sockets, thus reducing the risk of these hazards and protecting the safety of the user.

Additionally, in at least another embodiment of the present invention, as seen in Figures 94 and 95, an adaptor may be provided so that lamp bases may be retrofitted to couple with new sockets. For example, in Figure 94, lamp base 400 is a simple lamp base with four pin connectors 450, 452. Adaptor 410 can be fitted onto the end of lamp base 400. Adaptor 410 has a flat face 415, and end face 410 that is perpendicular to the flat face 415, and a key recess 440. After the adaptor 410 is fitted to lamp base 400, adaptor 410 simulates a step portion and pin connector 450 extends through adaptor 410. A second adaptor 410 can be also be fitted so that the lamp base 400 has two step portions. The key recesses 440 an the adaptors can be configured to match the key protrusions of a corresponding socket. In this way, it is possible to achieve the benefits of ensuring proper alignment when coupling a lamp base and socket, as described above, by simply upgrading already existing lamp bases with adaptors 410.

The presently disclosed embodiments are therefore to be considered in all respects as illustrative and not restrictive, the scope of the invention being indicated by the appended claims, rather than the foregoing description, and all changes which come -within the meaning and range of equivalency of the claims are therefore intended to be embraced therein.

## Claims

1. A lamp base (200) to be fitted on an end of a lamp bulb and designed to couple with a socket (300), the lamp base (200) comprising:
a cylindrical body having an end surface (202);
a first step portion and a second step portion, each comprising a flat face (220b,222b) extending perpendicular to the end surface (202), and a raised face (220c,222c) that is perpendicular to the flat face (220b, 222b);
a first upper pin connector (212) provided on the raised face (220c) of the first step portion and extending perpendicular to the first step portion;
a second upper pin connector (214) provided on the raised face (222c) of the second step portion and extending perpendicular to the first step portion;
a first (216) and second (218) lower pin connector provided on the end surface (202) and extending perpendicular to the end surface (202);
wherein the lamp base (200) is configured to couple with a socket (300) by linearly inserting the pin connectors (212, 214, 216, 218) into corresponding connectors on the socket, **characterized in that**
the lamp base (300) further comprises a first key recess (240) provided in the flat face (220b) of the first step portion, the first key recess (240) being structured such that when the lamp base (200) is coupled with the socket (300), the first key recess (240) is aligned with a first key protrusion (340) of the socket (300).

2. The lamp base (200) of claim 1, further comprising a second key recess (240) provided in the flat face (222b) of the second step portion.

3. The lamp base (200) of claim 1, wherein the first (216) and second (218) lower pin connectors are positioned symmetrically with respect to a center of the end surface (202).

4. The lamp base (200) of claim 1, wherein the first (216) and second (218) lower pin connectors are positioned asymmetrically with respect to a center of the end surface (202).

5. The lamp base (200) of claim 1, wherein the first step portion further comprises:
a first truncated portion (232) provided at a first end of the first step portion; and
a second truncated portion (234) provided at a second end of the first step portion.

6. The lamp base (200) of claim 1, wherein the first step portion comprises a first truncated portion (232) provided at a first end of the first step portion; and
the second step portion comprises a first second truncated portion (234) provided at a first end of the first second step portion.

7. A socket (300) designed to couple with a lamp base (200) comprising:
a cylindrical body having an end surface;
a central step portion (310) extending perpendicular to the end surface, the central step portion (310) comprising:
a first flat face (314) extending perpendicular to the end surface; and
a second flat face (316) extending perpendicular to the end surface;
a first upper connector (326) provided within a recess formed in the central step portion (310);
a second upper connector (328) provided within a recess formed in the central step portion (310);
a first lower connector (322) provided within a recess formed in the end surface;
a second lower connector (324) provided within a recess formed in the end surface; and
wherein the socket (300) is configured to couple with the lamp base (200) by linearly receiving pin connectors (212, 214, 216, 218) of the lamp base (200) into the corresponding connectors (322, 324, 326, 328),
**characterized in that** the socket (300) comprises a first key protrusion (340) protruding from the first flat face (314), the first key protrusion (340) being structured such that when socket (300) is coupled with the lamp base (200), the first key protrusion (340) is aligned with a first key recess (240) of the lamp base (200).

8. The socket (300) of claim 7, further comprising a second key protrusion (340) protruding from the second flat face (316).

9. The socket (300) of claim 7, wherein the first upper connector (326) and second upper connector (328) are positioned symmetrically with respect to a center of the end surface.

10. The socket (300) of claim 7, wherein the first upper connector (326) and the second upper connector (328) are positioned asymmetrically with respect to a center of the end surface.

11. The socket (300) of claim 7, further comprising:
a first tapered portion (312a) extending outward from a first end of the first flat face (314); and
a second tapered portion (312b) extending outward from a second end of the first flat face (314).

12. The socket (300) of claim 7, further comprising:
a first tapered portion (312a) extending outward from a first end of the first flat face (314); and
a second tapered portion (312b) extending outward from a first end of the second flat face (316).

13. A lamp base (200) and socket (300) assembly for use with a lamp bulb, the assembly comprising:
a lamp base (200) structured to fit on an end of a lamp bulb, the lamp bulb base (200) comprising:
a cylindrical lamp base body having a base end surface (202);
a first step portion and a second step portion, each comprising a flat face (220b,222b) extending perpendicular to the base end surface (202), and a raised face (220c, 222c) that is perpendicular to the flat face (220b, 222b);
a first upper pin connector (212) provided on the raised face (220c) of the first step portion and extending perpendicular to the first step portion;
a second upper pin connector (214) provided on the raised face (222c) of the second step portion and extending perpendicular to the first step portion; and
a first (216) and second (218) lower pin connector provided on the base end surface (202) and extending perpendicular to the end surface (202); and
a socket (300) structured to couple with the lamp base, the socket (300) comprising:
a cylindrical socket body having a socket end surface;
a central step portion (310) extending perpendicular to the socket end surface, the central step portion (310) comprising a first flat face (314) extending perpendicular to the end surface; and a second flat face (316) extending perpendicular to the end surface;
a first upper connector (326) provided within a recess formed in the central step portion (310);
a second upper connector (328) provided within a recess formed in the central step portion (310); a first lower connector (322) provided within a recess formed in the end surface; and
a second lower connector (324) provided within a recess formed in the end surface;
wherein the first lower connector (322) and second lower connector (324) are structured to couple with the first upper pin connector (212) and the second upper pin connector (214), by linearly inserting the first upper pin connector (212) and the second upper pin connector (214) into the first lower connector (322) and the second lower connector (324) respectively;
the first upper connector (326) and the second upper connector (328) are structured to couple with the first lower pin connector (216) and the second lower pin connector (218) by linearly inserting the first lower pin connector (216) and the second lower pin connector (218) into the first upper connector (326) and the second upper connector (328) respectively;
the central step portion (310) is structured to fit between the first step portion and the second step portion when the socket (300) and the lamp base (200) are coupled; and
the assembly further being **characterized by**
a first key recess (240) provided in the flat face (220b) of the first step portion and
a first key protrusion (340) protruding from the first flat face (314);
and in that the first key protrusion (340) is structured to align with the first key recess (240) when the socket (300) and lamp base (200) are coupled together.

14. The assembly of claim 13, further comprising:
a second key recess (240) provided in the flat surface (222b) of the second step portion;
a second key protrusion (340) protruding from the second flat face (316);
wherein the second key protrusion (340) is structured to align with the second key recess (240) when the socket (300) and lamp base (200) are coupled together.

15. The assembly of claim 13, wherein
the first step portion further comprises a first truncated portion (232) provided at first end of the first step portion, and a second truncated portion (234) provided at a second end of the first step portion;
a first tapered portion (312a) extends outward from a first end of the first flat face (314);
a second tapered portion (312b) extends outward from a second end of the first flat face (314); and
the first tapered portion (312a) aligns with the first truncated portion (232) and the second tapered portion (312b) aligns with the second truncated portion (234) when the socket (300) is coupled with the lamp base (200).

16. The assembly of claim 13, wherein
the first step portion further comprises a first truncated portion (232) provided at a first end of the first step portion; the second step portion further comprises a second truncated portion (234) provided at a first end of the second first step portion;
a first tapered portion (312a) extends outward from a first end of the first flat face;
a second tapered portion (312b) extends outward from a first end of the second flat face;and
the first tapered portion (312a) aligns with the first truncated portion (232) and the second tapered portion (312b) aligns with the second truncated portion (234) when the socket (300) is coupled with the lamp base (200).

## Patentansprüche

1. Lampensockel (200), der an ein Ende eines Lampenkolbens montierbar ist und ausgebildet ist, mit einer Fassung (300) verbindbar zu sein, wobei der Lampensockel (200) aufweist:
einen zylinderförmigen Körper, der eine Endfläche (202) aufweist;
einen ersten Stufenabschnitt und einen zweiten Stufenabschnitt, die jeweils eine flache Fläche (220b, 222b) aufweisen, die sich rechtwinklig zu der Endfläche (202) erstreckt, und eine erhöhte Fläche (220c, 222c), die rechtwinklig zu der flachen Fläche (220b, 222b) ist;
ein erster oberer Stiftverbinder (212), der an der erhöhten Fläche (220c) des ersten Stufenabschnitts vorgesehen ist und sich rechtwinklig zu dem ersten Stufenabschnitt erstreckt;
ein zweiter oberer Stiftverbinder (214), der an der erhöhten Fläche (222c) des zweiten Stufenabschnitts vorgesehen ist und sich rechtwinklig zu dem ersten Stufenabschnitt erstreckt;
ein erster (216) und ein zweiter (218) unterer Stiftverbinder, die an der Endfläche (202) vorgesehen sind und sich rechtwinklig zu der Endfläche (202) erstrecken;
wobei der Lampensockel (200) ausgelegt ist, mit einer Fassung (300) durch lineares Einfügen der Stiftverbinder (212, 214, 216, 218) in korrespondierende Anschlussbuchsen an der Fassung verbindbar zu sein, **dadurch gekennzeichnet, dass**
der Lampensockel (300) ferner eine erste Schlüsselvertiefung (240) aufweist, die in der flachen Fläche (220b) des ersten Stufenabschnitts vorgesehen ist, wobei die erste Schlüsselvertiefung (240) derart aufgebaut ist, dass, wenn der Lampensockel (200) mit der Fassung (300) verbunden wird, die erste Schlüsselvertiefung (240) mit einem ersten Schlüsselvorsprung (340) der Fassung (300) fluchtet.

2. Lampensockel (200) nach Anspruch 1, ferner aufweisend eine zweite Schlüsselvertiefung (240), die in der flachen Fläche (222b) des zweiten Stufenabschnitts vorgesehen ist.

3. Lampensockel (200) nach Anspruch 1, wobei der erste (216) und der zweite (218) untere Stiftverbinder bezüglich einer Mitte der Endfläche (202) symmetrisch platziert sind.

4. Lampensockel (200) nach Anspruch 1, wobei der erste (216) und der zweite (218) untere Stiftverbinder bezüglich einer Mitte der Endfläche (202) asymmetrisch platziert sind.

5. Lampensockel (200) nach Anspruch 1, wobei der erste Stufenabschnitt ferner aufweist:
einen ersten angeschnittenen Abschnitt (232), der an einem ersten Ende des ersten Stufenabschnitts vorgesehen ist; und
einen zweiten angeschnittenen Abschnitt (234), der an einem zweiten Ende des ersten Stufenabschnitts vorgesehen ist.

6. Lampensockel (200) nach Anspruch 1, wobei der erste Stufenabschnitt einen ersten angeschnittenen Abschnitt (232) aufweist, der an einem ersten Ende des ersten Stufenabschnitts vorgesehen ist; und der zweite Stufenabschnitt einen ersten zweiten angeschnittenen Abschnitt (234) aufweist, der an einem ersten Ende des ersten zweiten Stufenabschnitts vorgesehen ist.

7. Fassung (300), die ausgebildet ist, mit einem Lampensockel (200) verbindbar zu sein, aufweisend:
einen zylinderförmigen Körper, der eine Endfläche aufweist;
einen mittigen Stufenabschnitt (310), der sich rechtwinklig zu der Endfläche erstreckt, wobei der mittige Stufenabschnitt (310) aufweist:
eine erste flache Fläche (314), die sich rechtwinklig zu der Endfläche erstreckt; und
eine zweite flache Fläche (316), die sich rechtwinklig zu der Endfläche erstreckt;
eine erste obere Anschlussbuchse (326), die in einer Vertiefung vorgesehen ist, die in dem mittigen Stufenabschnitt (310) gebildet ist;
eine zweite obere Anschlussbuchse (328), die in einer Vertiefung vorgesehen ist, die in dem mittigen Stufenabschnitt (310) gebildet ist;
eine erste untere Anschlussbuchse (322), die in einer Vertiefung vorgesehen ist, die in der Endfläche gebildet ist;
eine zweite untere Anschlussbuchse (324), die in einer Vertiefung vorgesehen ist, die in der Endfläche gebildet ist; und
wobei die Fassung (300) ausgelegt ist, mit dem Lampensockel (200) durch lineares Aufnehmen von Stiftverbindern (212, 214, 216, 218) des Lampensockels (200) in die korrespondierenden Anschlussbuchsen (322, 324, 326, 328) verbindbar zu sein,
**dadurch gekennzeichnet, dass** die Fassung (300) einen ersten Schlüsselvorsprung (340) aufweist, der von der ersten flachen Fläche (314) vorsteht, wobei der erste Schlüsselvorsprung (340) derart aufgebaut ist, dass, wenn die Fassung (300) mit dem Lampensockel (200) verbunden wird, der erste Schlüsselvorsprung (340) mit einer ersten Schlüsselvertiefung (240) des Lampensockels (200) fluchtet.

8. Fassung (300) nach Anspruch 7, ferner aufweisend einen zweiten Schlüsselvorsprung (340), der von der zweiten flachen Fläche (316) vorsteht.

9. Fassung (300) nach Anspruch 7, wobei die erste obere Anschlussbuchse (326) und die zweiten obere Anschlussbuchse (328) bezüglich einer Mitte der Endfläche symmetrisch platziert sind.

10. Fassung (300) nach Anspruch 7, wobei die erste obere Anschlussbuchse (326) und die zweite obere Anschlussbuchse (328) bezüglich einer Mitte der Endfläche asymmetrisch platziert sind.

11. Fassung (300) nach Anspruch 7, ferner aufweisend:
einen ersten angeschrägten Abschnitt (312a), der sich von einem ersten Ende der ersten flachen Fläche (314) nach außen erstreckt; und
einen zweiten angeschrägten Abschnitt (312b), der sich von einem zweiten Ende der ersten flachen Fläche (314) nach außen erstreckt.

12. Fassung (300) nach Anspruch 7, ferner aufweisend:
einen ersten angeschrägten Abschnitt (312a), der sich von einem ersten Ende der ersten flachen Fläche (314) nach außen erstreckt; und
einen zweiten angeschrägten Abschnitt (312b), der sich von einem ersten Ende der zweiten flachen Fläche (316) nach außen erstreckt

13. Anordnung aus einem Lampensockel (200) und einer Fassung (300) zur Verwendung mit einem Lampenkolben, wobei die Anordnung aufweist:
einen Lampensockel (200), der aufgebaut ist, auf ein Ende eines Lampenkolbens zu passen, wobei der Lampensockel (200) aufweist:
einen zylinderförmigen Lampensockelkörper, der eine Sockelendfläche (202) aufweist;
einen ersten Stufenabschnitt und einen zweiten Stufenabschnitt, die jeweils eine flache Fläche (220b, 222b) aufweisen, die sich rechtwinklig zu der Sockelendfläche (202) erstreckt, und eine erhöhte Fläche (220c, 222c), die rechtwinklig zu der flachen Fläche (220b, 222b) ist;
ein erster oberer Stiftverbinder (212), der an der erhöhten Fläche (220c) des ersten Stufenabschnitts vorgesehen ist und sich rechtwinklig zu dem ersten Stufenabschnitt erstreckt;
ein zweiter oberer Stiftverbinder (214), der an der erhöhten Fläche (222c) des zweiten Stufenabschnitts vorgesehen ist und sich rechtwinklig zu dem ersten Stufenabschnitt erstreckt; und
ein erster (216) und ein zweiter (218) unterer Stiftverbinder, die an der Sockelendfläche (202) vorgesehen sind und sich rechtwinklig zu der Endfläche (202) erstrecken; und
eine Fassung (300), die aufgebaut ist, mit dem Lampensockel verbindbar zu sein, wobei die Fassung (300) aufweist:
einen zylinderförmigen Fassungskörper, der eine Fassungsendfläche aufweist;
einen mittigen Stufenabschnitt (310), der sich rechtwinklig zu der Fassungsendfläche erstreckt, wobei der mittige Stufenabschnitt (310) eine erste flache Fläche (314) aufweist, die sich rechtwinklig zu der Endfläche erstreckt; und eine zweite flache Fläche (316), die sich rechtwinklig zu der Endfläche erstreckt;
eine erste obere Anschlussbuchse (326), die in einer Vertiefung vorgesehen ist, die in dem mittigen Stufenabschnitt (310) gebildet ist;
eine zweite obere Anschlussbuchse (328), die in einer Vertiefung vorgesehen ist, die in dem mittigen Stufenabschnitt (310) gebildet ist;
eine erste untere Anschlussbuchse (322), die in einer Vertiefung vorgesehen ist, die in der Endfläche gebildet ist; und
eine zweite untere Anschlussbuchse (324), die in einer Vertiefung vorgesehen ist, die in der Endfläche gebildet ist;
wobei die erste untere Anschlussbuchse (322) und die zweite untere Anschlussbuchse (324) aufgebaut sind, mit dem ersten oberen Stiftverbinder (212) und dem zweiten oberen Stiftverbinder (214) durch lineares Einfügen des ersten oberen Stiftverbinders (212) und des zweiten oberen Stiftverbinders (214) in die erste untere Anschlussbuchse (322) beziehungsweise die zweite untere Anschlussbuchse (324) verbindbar zu sein;
wobei die erste obere Anschlussbuchse (326) und die zweite obere Anschlussbuchse (328) aufgebaut sind, mit dem ersten unteren Stiftverbinder (216) und dem zweiten unteren Stiftverbinder (218) durch lineares Einfügen des ersten unteren Stiftverbinders (216) und des zweiten unteren Stiftverbinders (218) in die erste obere Anschlussbuchse (326) beziehungsweise die zweite obere Anschlussbuchse (328) verbindbar zu sein;
wobei der mittige Stufenabschnitt (310) aufgebaut ist, zwischen den ersten Stufenabschnitt und den zweiten Stufenabschnitt zu passen, wenn die Fassung (300) und der Lampensockel (200) verbunden sind; und
wobei die Anordnung ferner **gekennzeichnet ist durch**
eine erste Schlüsselvertiefung (240), die in der flachen Fläche (220b) des ersten Stufenabschnitts vorgesehen ist, und
einen ersten Schlüsselvorsprung (340), der von der ersten flachen Fläche (314) vorsteht;
und **dadurch**, dass der erste Schlüsselvorsprung (340) aufgebaut ist, mit der ersten Schlüsselvertiefung (240) zu fluchten, wenn die Fassung (300) und der Lampensockel (200) miteinander verbunden sind.

14. Anordnung nach Anspruch 13, ferner aufweisend:
eine zweite Schlüsselvertiefung (240), die in der flachen Fläche (222b) des zweiten Stufenabschnitts vorgesehen ist;
und ein zweiter Schlüsselvorsprung (340), der von der zweiten flachen Fläche (316) vorsteht;
wobei der zweite Schlüsselvorsprung (340) aufgebaut ist, mit der zweiten Schlüsselvertiefung (240) zu fluchten, wenn die Fassung (300) und der Lampensockel (200) miteinander verbunden sind.

15. Anordnung nach Anspruch 13, wobei
der erste Stufenabschnitt ferner einen ersten angeschnittenen Abschnitt (232) aufweist, der an einem ersten Ende des ersten Stufenabschnitts vorgesehen ist, und einen zweiten angeschnittenen Abschnitt (234), der an einem zweiten Ende des ersten Stufenabschnitts vorgesehen ist;
sich ein erster angeschrägter Abschnitt (312a) von einem ersten Ende der ersten flachen Fläche (314) nach außen erstreckt;
sich ein zweiter angeschrägter Abschnitt (312b) von einem zweiten Ende der ersten flachen Fläche (314) nach außen erstreckt; und
der erste angeschrägte Abschnitt (312a) mit dem ersten angeschnittenen Abschnitt (232) fluchtet und der zweite angeschrägte Abschnitt (312b) mit dem zweiten angeschnittenen Abschnitt (234) fluchtet, wenn die Fassung (300) mit dem Lampensockel (200) verbunden ist.

16. Anordnung nach Anspruch 13, wobei
der erste Stufenabschnitt ferner einen ersten angeschnittenen Abschnitt (232) aufweist, der an einem ersten Ende des ersten Stufenabschnitts vorgesehen ist; der zweite Stufenabschnitt ferner einen zweiten angeschnittenen Abschnitt (234) aufweist, der an einem ersten Ende des zweiten ersten Stufenabschnitts vorgesehen ist;
sich ein erster angeschrägter Abschnitt (312a) von einem ersten Ende der ersten flachen Fläche nach außen erstreckt;
sich ein zweiter angeschrägter Abschnitt (312b) von einem ersten Ende der zweiten flachen Fläche nach außen erstreckt; und
der erste angeschrägte Abschnitt (312a) mit dem ersten angeschnittenen Abschnitt (232) fluchtet und der zweite angeschrägte Abschnitt (312b) mit dem zweiten angeschnittenen Abschnitt (234) fluchtet, wenn die Fassung (300) mit dem Lampensockel (200) verbunden ist.

## Revendications

1. Culot de lampe (200) destiné à être monté sur une extrémité d'une ampoule de lampe et conçu pour être couplé à une douille (300), le culot de lampe (200) comprenant:
un corps cylindrique ayant une surface d'extrémité (202);
une première portion formant gradin et une deuxième portion formant gradin présentant chacune une face plane (220b, 222b) qui s'étend perpendiculairement à la surface d'extrémité (202) et une face surélevée (220c, 222c) qui est perpendiculaire à la face plane (220b, 222b);
un premier connecteur à broche (212) supérieur qui est prévu sur la face surélevée (220c) de la première portion formant gradin et qui s'étend perpendiculairement à la première portion formant gradin;
un deuxième connecteur à broche (214) supérieur qui est prévu sur la face surélevée (222c) de la deuxième portion formant gradin et qui s'étend perpendiculairement à la première portion formant gradin;
des premier (216) et deuxième (218) connecteurs à broche inférieurs qui sont prévus sur la surface d'extrémité (202) et s'étendent perpendiculairement à la surface d'extrémité (202);
dans lequel le culot de lampe (200) est configuré pour être couplé à une douille (300) en insérant de façon linéaire les connecteurs à broche (212, 214, 216, 218) dans des prises correspondantes sur la douille, **caractérisé par le fait que**
le culot de lampe (200) comprend en outre un premier creux clé (240) qui est prévu dans la face plane (220b) de la première portion formant gradin, ledit premier creux clé (240) étant configuré de manière à ce que, lorsque le culot de lampe (200) est couplé à la douille (300), le premier creux clé (240) soit aligné avec une première projection clé (340) de la douille (300).

2. Culot de lampe (200) selon la revendication 1, comprenant en outre un deuxième creux clé (240) qui est prévu dans la face plane (222b) de la deuxième portion formant gradin.

3. Culot de lampe (200) selon la revendication 1, dans lequel les premier (216) et deuxième (218) connecteurs à broche inférieurs sont positionnés de façon symétrique par rapport à un centre de la surface d'extrémité (202).

4. Culot de lampe (200) selon la revendication 1, dans lequel les premier (216) et deuxième (218) connecteurs à broche inférieurs sont positionnés de façon asymétrique par rapport à un centre de la surface d'extrémité (202).

5. Culot de lampe (200) selon la revendication 1, dans lequel la première portion formant gradin comprend en outre:
une première portion tronquée (232) qui est prévue à une première extrémité de la première portion formant gradin; et
une deuxième portion tronquée (234) qui est prévue à une deuxième extrémité de la première portion formant gradin.

6. Culot de lampe (200) selon la revendication 1, dans lequel la première portion formant gradin comprend une première portion tronquée (232) qui est prévue à une première extrémité de la première portion formant gradin; et
ladite deuxième portion formant gradin présente une première deuxième portion tronquée (234) qui est prévue à une première extrémité de la première deuxième portion formant gradin.

7. Douille (300) conçue pour être couplée à un culot de lampe (200), comprenant:
un corps cylindrique présentant une surface d'extrémité;
une portion centrale formant gradin (310) qui s'étend perpendiculairement à la surface d'extrémité, ladite portion centrale formant gradin (310) comprenant:
une première face plane (314) qui s'étend perpendiculairement à la surface d'extrémité; et
une deuxième face plane (316) qui s'étend perpendiculairement à la surface d'extrémité;
une première prise supérieure (326) prévue dans un creux ménagé dans ladite portion centrale formant gradin (310);
une deuxième prise supérieure (328) qui est prévue dans un creux ménagé dans ladite portion centrale formant gradin (310);
une première prise inférieure (322) qui est prévue dans un creux ménagé dans la surface d'extrémité;
une deuxième prise inférieure (324) qui est prévue dans un creux ménagé dans la surface d'extrémité; et
dans laquelle ladite douille (300) est configurée pour être couplée au culot de lampe (200) en recevant de façon linéaire des connecteurs à broche (212, 214, 216, 218) du culot de lampe (200) dans les prises (322, 324, 326, 328) correspondantes,
**caractérisée par le fait que** la douille (300) présente une première projection clé (340) qui fait saillie de la première face plane (314), la première projection clé (340) étant configurée de manière à ce que, lorsque la douille (300) est couplée au culot de lampe (200), la première projection clé (340) soit alignée avec un premier creux clé (240) du culot de lampe (200).

8. Douille (300) selon la revendication 7, comprenant en outre une deuxième projection clé (340) qui fait saillie de la deuxième face plane (316).

9. Douille (300) selon la revendication 7, dans laquelle la première prise supérieure (326) et la deuxième prise supérieure (328) sont positionnées de façon symétrique par rapport à un centre de la surface d'extrémité.

10. Douille (300) selon la revendication 7, dans laquelle la première prise
supérieure (326) et la deuxième prise supérieure (328) sont positionnées de façon asymétrique par rapport à un centre de la surface d'extrémité.

11. Douille (300) selon la revendication 7, comprenant en outre:
une première portion biseautée (312a) qui s'étend vers l'extérieur à partir d'une première extrémité de la première face plane (314); et
une deuxième portion biseautée (312b) qui s'étend vers l'extérieur à partir d'une deuxième extrémité de la première face plane (314).

12. Douille (300) selon la revendication 7, comprenant en outre:
une première portion biseautée (312a) qui s'étend vers l'extérieur à partir d'une première extrémité de la première face plane (314); et
une deuxième portion biseautée (312b) qui s'étend vers l'extérieur à partir d'une première extrémité de la deuxième face plane (316).

13. Ensemble de culot de lampe (200) et de douille (300) destiné à être utilisé avec une ampoule de lampe, ledit ensemble comprenant:
un culot de lampe (200) configuré pour s'ajuster sur une extrémité d'une ampoule de lampe, ledit culot de lampe (200) comprenant:
un corps cylindrique de culot de lampe qui présente une surface d'extrémité de culot (202);
une première portion formant gradin et une deuxième portion formant gradin présentant chacune une face plane (220b, 222b) qui s'étend perpendiculairement à la surface d'extrémité de culot (202) et une face surélevée (220c, 222c) qui est perpendiculaire à la face plane (220b, 222b);
un premier connecteur à broche (212) supérieur qui est prévu sur la face surélevée (220c) de la première portion formant gradin et qui s'étend perpendiculairement à la première portion formant gradin;
un deuxième connecteur à broche (214) supérieur qui est prévu sur la face surélevée (222c) de la deuxième portion formant gradin et qui s'étend perpendiculairement à la première portion formant gradin; et
des premier (216) et deuxième (218) connecteurs à broche inférieurs qui sont prévus sur la surface d'extrémité de culot (202) et s'étendent perpendiculairement à la surface d'extrémité (202); et
une douille (300) conçue pour être couplée au culot de lampe, ladite douille (300) comprenant:
un corps cylindrique de douille présentant une surface d'extrémité de douille;
une portion centrale formant gradin (310) qui s'étend perpendiculairement à la surface d'extrémité de douille, ladite portion centrale formant gradin (310) comprenant une première face plane (314) qui s'étend de façon perpendiculaire à la surface d'extrémité; et une deuxième face plane (316) qui s'étend de façon perpendiculaire à la surface d'extrémité;
une première prise supérieure (326) qui est prévue dans un creux ménagé dans ladite portion centrale formant gradin (310);
une deuxième prise supérieure (328) qui est prévue dans un creux ménagé dans ladite portion centrale formant gradin (310);
une première prise inférieure (322) qui est prévue dans un creux ménagé dans la surface d'extrémité; et
une deuxième prise inférieure (324) qui est prévue dans un creux ménagé dans la surface d'extrémité;
dans lequel la première prise inférieure (322) et la deuxième prise inférieure (324) sont conçues de manière à être couplées au premier connecteur à broche (212) supérieur et au deuxième connecteur à broche (214) supérieur en insérant de façon linéaire le premier connecteur à broche (212) supérieur et le deuxième connecteur à broche (214) supérieur dans la première prise inférieure (322) et dans la deuxième prise inférieure (324) respectivement;
dans lequel la première prise supérieure (326) et la deuxième prise supérieure (328) sont conçues de manière à être couplées au premier connecteur à broche (216) inférieur et au deuxième connecteur à broche (218) inférieur en insérant de façon linéaire le premier connecteur à broche (216) inférieur et le deuxième connecteur à broche (218) inférieur dans la première prise supérieure (326) et dans la deuxième prise supérieure (328) respectivement;
dans lequel ladite portion centrale formant gradin (310) est conçue pour s'ajuster entre la première portion formant gradin et la deuxième portion formant gradin lorsque la douille (300) et le culot de lampe (200) sont couplés entre eux; et
ledit ensemble étant **caractérisé en outre par**
un premier creux clé (240) qui est prévu dans la face plane (220b) de la première portion formant gradin et
une première projection clé (340) qui fait saillie de la première face plane (314);
et par le fait que la première projection clé (340) est configurée pour être alignée avec le premier creux clé (240) lorsque la douille (300) et le culot de lampe (200) sont couplés entre eux.

14. Ensemble selon la revendication 13, comprenant en outre:
un deuxième creux clé (240) qui est prévu dans la face plane (222b) de la deuxième portion formant gradin; et
une deuxième projection clé (340) qui fait saillie de la deuxième face plane (316);
dans lequel ladite deuxième projection clé (340) est configurée pour être alignée avec le deuxième creux clé (240) lorsque la douille (300) et le culot de lampe (200) sont couplés entre eux.

15. Ensemble selon la revendication 13, dans lequel
la première portion formant gradin comprend en outre une première portion tronquée (232) qui est prévue à une première extrémité de la première portion formant gradin, et une deuxième portion tronquée (234) qui est prévue à une deuxième extrémité de la première portion formant gradin; une première portion biseautée (312a) s'étend vers l'extérieur à partir d'une première extrémité de la première face plane (314);
une deuxième portion biseautée (312b) s'étend vers l'extérieur à partir d'une deuxième extrémité de la première face plane (314); et
la première portion biseautée (312a) est alignée avec la première portion tronquée (232) et la deuxième portion biseautée (312b) est alignée avec la deuxième portion tronquée (234) lorsque la douille (300) est couplée au culot de lampe (200).

16. Ensemble selon la revendication 13, dans lequel
la première portion formant gradin comprend en outre une première portion tronquée (232) qui est prévue à une première extrémité de la première portion formant gradin; la deuxième portion formant gradin comprend en outre une deuxième portion tronquée (234) qui est prévue à une première extrémité de la deuxième première portion formant gradin;
une première portion biseautée (312a) s'étend vers l'extérieur à partir d'une première extrémité de la première face plane;
une deuxième portion biseautée (312b) s'étend vers l'extérieur à partir d'une première extrémité de la deuxième face plane; et
la première portion biseautée (312a) est alignée avec la première portion tronquée (232) et la deuxième portion biseautée (312b) est alignée avec la deuxième portion tronquée (234) lorsque la douille (300) est couplée au culot de lampe (200).
